# FASCICULE DE BREVET EUROPEEN

(11) **EP 2 240 226 B1**
(45) Date de publication et mention de la délivrance du brevet: **09.05.2018**
(21) Numéro de dépôt: 08863083.5
(22) Date de dépôt: 02.12.2008
(51) Int. Cl.: A61M 15/00

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING FLUID PRODUCT

(30) Priorité: 03.12.2007 FR 0759520
(43) Date de publication de la demande: 20.10.2010
(73) Titulaire: Aptar France SAS, 27110 Le Neubourg (FR)
(72) Inventeur: SALLAK, Zakaria, F-76100 Rouen (FR)
(74) Mandataire: CAPRI
(86) Numéro de dépôt international: PCT/FR2008/052180
(87) Numéro de publication internationale: WO 2009/077701

(56) Documents cités:
- WO-A-2006/079749
- WO-A2-2008/012458

## Description

La présente invention concerne un dispositif de distribution de produit fluide, et plus particulièrement un inhalateur de poudre sèche.

Les inhalateurs de poudre sèche sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blister allongé ou des blisters disposés sur un disque circulaire rotatif. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre évidemment, l'utilisation est plus complexe, puisque l'utilisateur est obligé de charger une capsule dans l'inhalateur avant chaque utilisation. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a changé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. En variante, il a été proposé de percer la paroi ou couche de fermeture. Ceci peut présenter l'inconvénient que les parties de paroi découpées risquent de retenir une partie de la dose à l'intérieur du réservoir, la précision et reproductibilité de dosage n'étant donc pas garanties. Par ailleurs, il est connu d'utiliser un compteur ou indicateur de doses pour informer l'utilisateur du nombre de doses distribuées ou restant à distribuer. Un inconvénient classique avec ces compteurs est que soit ils sont très encombrants, augmentant d'autant la dimension de l'inhalateur lui-même, soit l'affichage est très petit et souvent difficile à lire, notamment pour les personnes âgées. Ceci est notamment vrai pour les compteurs destinés à compter un nombre élevé de doses, par exemple 60 doses.

Le document WO 2006/079749 décrit un dispositif de l'art antérieur.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un dispositif qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir un dispositif qui évite tout risque de sous-dosage, l'ouverture du réservoir, l'expulsion de la dose et le comptage de la dose émise ne se produisant qu'en cas d'inhalation de l'utilisateur. De plus, la présente invention a pour but d'éviter tout risque de perte de dose en l'absence d'inhalation même si l'utilisateur manipule le dispositif.

La présente invention a encore pour but de fournir un dispositif permettant de compter le nombre de doses émises ou restant à émettre qui soit de dimensions raisonnables tout en proposant un affichage aisément lisible par les utilisateurs.

La présente invention a donc pour objet un dispositif de distribution de produits fluides selon la revendication 1.

Des modes de réalisation avantageux sont décrits dans les revendications dépendantes.

Ces caractéristiques et avantages et d'autres de la présente invention apparaîtront plus clairement au cours de la description détaillée suivante de plusieurs modes et variantes de réalisation de celle-ci, faite en référence aux dessins joints, donnés à titres d'exemples non limitatifs, sur lesquels,
- la figure 1 est une vue schématique partiellement en section d'une variante d'un dispositif de distribution de produit fluide, en position armée, avant inhalation,
- la figure 2 est une vue similaire à la figure 1, en position après inhalation,
- la figure 3 est une vue schématique partiellement en section d'une autre variante d'un dispositif de distribution de produit fluide, en position après inhalation,
- la figure 4 est une vue similaire à la figure 3, en position armée, avant inhalation,
- la figure 5 est une vue schématique partiellement en section d'encore une autre variante d'un dispositif de distribution de produit fluide, avant armement,
- la figure 6 est une vue de détail de la figure 5,
- la figure 7 est une vue similaire à la figure 5, après armement et avant inhalation,
- la figure 8 est une vue de détail de la figure 7,
- la figure 9 est une vue similaire aux figures 5 et 7, après inhalation,
- la figure 10 est une schématique d'encore une autre variante d'un dispositif de distribution de produit fluide, en position après inhalation,
- la figure 11 est une vue similaire à la figure 10, après retour en position de repos,
- la figure 12 est une vue schématique en perspective d'un détail des figures 10 et 11,
- la figure 13 est une vue schématique en section d'une partie d'un compteur avantageux, vue de derrière,
- la figure 14 est une vue partiellement en section de la partie de compteur de la figure 13, vue de devant,
- les figures 15 et 16 sont respectivement des vues de derrière et devant du premier élément de comptage rotatif,
- les figures 17a et 17b sont respectivement des vues partielles de derrière et devant du second élément de comptage rotatif,
- les figures 18 et 19 sont respectivement des vues partielles en section de l'élément d'actionnement, en position de repos et d'actionnement,
- la figure 20 est une vue schématique en section similaire à la figure 13, avec le doigt déformable déformé par la came, et
- la figure 21 est une vue de détail de la figure 20.

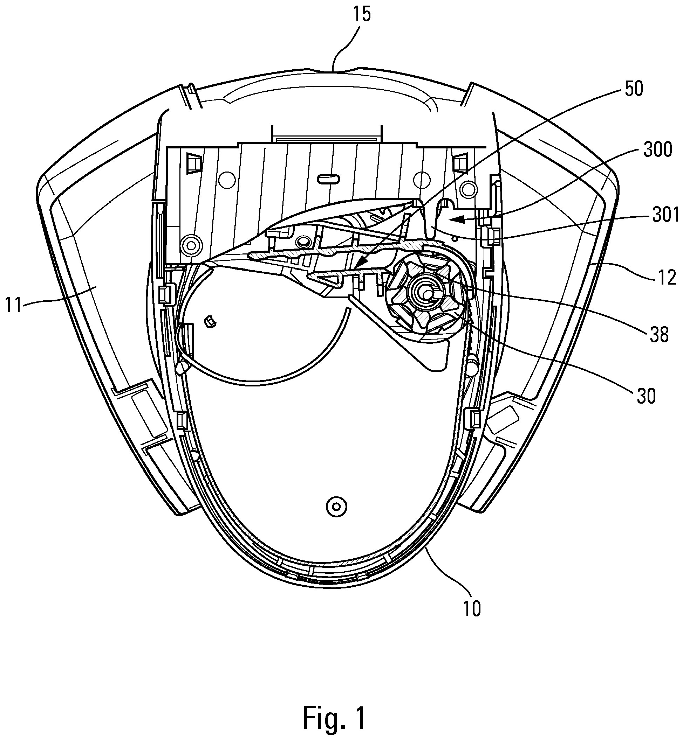

La présente invention concerne de préférence un inhalateur de poudre sèche. Diverses variantes sont représentées sur les figures, étant entendu que les caractéristiques représentées sur ces figures pourraient être combinées entre elles d'une quelconque manière appropriée. Les dessins ne sont donc pas limitatifs. Par ailleurs, de nombreuses caractéristiques de cet inhalateur pourraient ainsi être mise en oeuvre pour distribuer du liquide à la place de la poudre. L'inhalateur comporte un corps central 10 sur lequel sont montées coulissantes deux éléments ou ailes latérales 11, 12 formant capot lorsque le dispositif est fermé et adaptées à être séparées l'une de l'autre pour ouvrir le dispositif et ainsi charger le dispositif comme cela sera décrit ci-après. Le corps 10 peut être de forme environ arrondie en partie basse, et relativement plat en partie haute, comme représenté notamment sur la figure 1, mais il pourrait avoir tout autre forme appropriée. Le corps 10 comporte un orifice de distribution et d'inhalation 15 à travers lequel l'utilisateur va inhaler lors de l'actionnement du dispositif. Les deux parties latérales 11, 12 formant capot peuvent s'ouvrir par pivotement autour d'un axe de rotation commun, mais tout autre moyen d'ouverture du dispositif est envisageable. Par ailleurs, on pourrait ne prévoir qu'un seul élément de capot mobile par rapport au corps au lieu des deux représentés notamment sur la figure 1.

Avantageusement, le corps comporte une fenêtre 19 à travers laquelle le comptage des doses émises ou restant à émettre peut être affiché de manière visible pour l'utilisateur. Cette fenêtre peut par exemple être prévue sur ou proche de l'axe de rotation des éléments de capot 11, 12 formant le capot, mais elle pourrait être à un autre endroit. A l'intérieur du corps, il peut être prévu un support 20 de réservoirs individuels 21. Les réservoirs sont avantageusement du type blisters, et le support de réservoir est de préférence une bande souple allongée, sur laquelle les blisters sont disposés les uns derrière les autres, en nombres quelconques appropriés, de manière connue. La bande de blister peut avantageusement être constituée d'une couche ou paroi de base formant des cavités recevant les doses de poudre, et d'une couche ou paroi de fermeture qui obture de manière étanche chacun desdits blisters. La bande de blister peut être enroulée à l'intérieur du corps et des premiers moyens de déplacement de bande 30 sont prévus pour progressivement dérouler cette bande de blister et amener un réservoir individuel ou blister respectif dans une position de distribution à chaque actionnement du dispositif. Lorsqu'un réservoir individuel a été vidé par une inhalation, la partie de bande comportant lesdits réservoirs vides est avantageusement adaptée à s'enrouler dans un autre endroit dudit corps 10.

Des moyens d'ouverture de réservoir 80 sont prévus dans ou solidaires du corps 10, ces moyens d'ouverture comportant des moyens de perçage et/ou de coupage de la couche de fermeture des blisters. Ces moyens d'ouverture ne sont représentés que schématiquement sur les figures.

Des moyens de support mobiles 50 sont adaptés à supporter au moins le réservoir qui est destiné à être ouvert lors de la prochaine inhalation. Ces moyens de supports mobiles 50 formant des seconds moyens de déplacement qui sont adaptés à déplacer le réservoir à vider contre lesdits moyens d'ouverture du dispositif lors de l'actionnement. Avantageusement, ces moyens de supports mobiles 50 sont sollicités directement ou indirectement par des moyens de chargement 800 comprenant un élément de chargement élastiquement déformable 51, tel qu'un ressort, une tige ou tout autre élément élastique équivalent, ledit élément de chargement étant préchargé, notamment lors de l'ouverture du dispositif. Avantageusement, les moyens de support mobiles 50 sont déplaçables entre une première position (position de non-distribution) et une seconde position (position de distribution) qui est la position d'ouverture de réservoir et donc d'inhalation.

Avantageusement, les moyens de support mobiles 50 comportent une pièce sensiblement rigide, telle qu'une tige, articulée par rapport audit corps 10. Une roue de guidage ou d'indexage 30 fixée de manière rotative sur lesdits moyens de support mobiles 50, reçoit et guide les blisters. Une rotation de cette roue de guidage 30 fait ainsi avancer la bande de blister dans une première direction. Dans une position angulaire particulière, un réservoir ou blister donné est toujours en position pour être ouvert par les moyens d'ouverture.

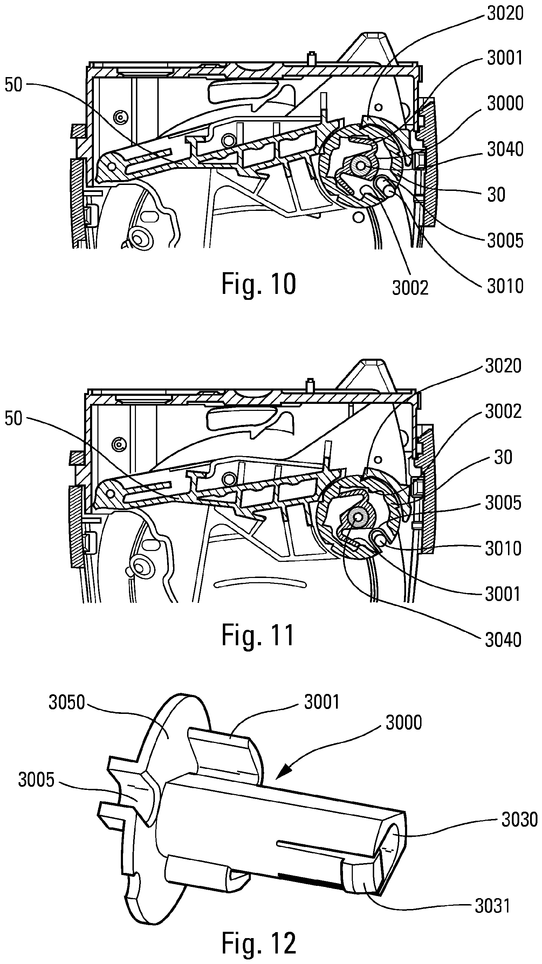

Cette roue d'indexage 30 forme donc les premiers moyens de déplacement des réservoirs 21, permettant de faire avancer la bande souple 20 hors de chaque cycle d'actionnement, alors que lesdits moyens de support mobiles 50 forment des records moyens de déplacement amenant un réservoir respectif contre lesdits moyens d'ouverture 80 à chaque actionnement. Les figures 10 à 12 représentent une variante avantageuse pour actionner ladite roue d'indexage 30 pour la faire tourner et ainsi faire avancer la bande de blisters 20. Dans cette variante, un organe d'actionnement 3000 est disposé de manière concentrique ou coaxiale, radialement à l'intérieur de la roue d'indexage. Cet organe d'actionnement 3000 est de préférence monté rotatif sur un plot 3040 solidaire des moyens de support mobiles 50. Il est rotatif par rapport à la roue d'indexage 30, et comporte au moins une, de préférence deux pattes flexibles 3001 adaptées à coopérer avec une denture interne 3002 de la roue d'indexage 30. En variante, l'organe d'actionnement 3000 pourrait comporter la denture et la roue d'indexage la ou les patte(s) flexible(s). L'organe d'actionnement 3000 comporte avantageusement aussi une gorge 3005 adaptée à coopérer avec une partie du corps 10, par exemple une projection 3010, éventuellement souple et/ou déformable. Ainsi, lorsque les moyens de support mobiles 50 se déplacent, entraînant avec eux l'organe d'actionnement 3000 et la roue d'indexage 30, la projection 3010 du corps qui reste fixe provoque la rotation de l'organe d'actionnement 3000, dans un premier sens lors du retour des moyens de support mobiles 50 vers leur position de repos, et dans un second sens inverse au premier sens, lorsque les moyens de support mobiles 50 se déplacent vers leur position de distribution. Bien entendu, l'inverse est aussi envisageable. Dans ledit premier sens, les doigts flexibles 3001 poussent dans la denture 3002 pour faire tourner la roue d'indexage 30, les moyens anti-retour 3020 glissant sur la pente inclinée avec laquelle ils coopèrent. Dans le second sens, les moyens anti-retour 3020 empêchent la rotation de la roue d'indexage 30, et les pattes flexibles glissent en se déformant sur les pattes inclinées des dents de la denture 3002. L'organe d'actionnement 3000 comporte de préférence un tube central creux 3030 monté sur le plot 3040, et autour duquel s'assemble la roue d'indexage 30, de préférence par encliquetage sur une patte d'encliquetage 3031 dudit tube. L'organe d'actionnement 3000 comporte aussi avantageusement un plateau de support axial 3050 formant butée axiale par la roue d'indexage 30 qui peut alors tourner sur ledit organe d'actionnement, en étant maintenue entre ledit plateau 3050 et ladite patte d'encliquetage 3031.

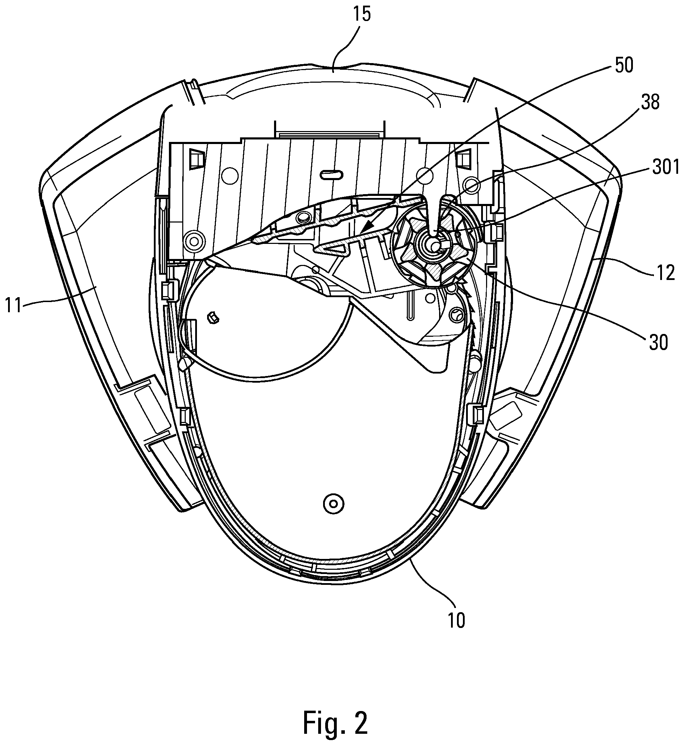

Selon l'invention, des moyens de positionnement ou de centrage 300 de ladite roue de guidage 30 sont prévus pour déterminer précisément la position angulaire de ladite roue de guidage 30 après chaque rotation. Ces moyens de positionnement 300 peuvent, selon une variante avantageuse, comporter une projection ou doigt 301 dont une extrémité coopère avec des moyens de réception complémentaires 38, tels que des encoches, prévus sur ladite roue de guidage 30. Selon l'invention, le doigt 301 est solidaire des moyens d'ouverture 80. Avantageusement, le doigt 301 comporte un profil tronconique environ en V qui guide automatiquement ledit doigt 301 dans l'encoche 38, assurant un positionnement angulaire précis à chaque rotation. Ces moyens de positionnement 300 sont visibles sur les figures 1 et 2. Ces moyens de positionnement 300 peuvent aussi déterminer la position de butée du réservoir 21 par rapport aux moyens d'ouverture 80. D'autres moyens de butée sont aussi envisageables. Un avantage de ce type de moyens de butée est aussi de bloquer brusquement le réservoir plein juste après qu'il ait été percé par l'aiguille 80, avec pour effet de secouer la poudre contenue dans le réservoir et aussi favoriser sa distribution dans le flux d'inhalation. De tels moyens de butée pourraient être utilisés indépendamment des moyens de positionnement 300.

Lorsque le réservoir se déplace vers sa position d'ouverture pour être ouvert par les moyens d'ouverture 80, ces moyens d'ouverture sont de préférence fixes par rapport au corps 10. Toutefois, il est envisageable que ces moyens d'ouverture puissent également être mobiles pendant la phase d'ouverture du réservoir. Par exemple, les moyens d'ouverture pourraient se déplacer en direction du réservoir pendant que le réservoir se déplace en direction des moyens d'ouverture. Dans une autre variante, on pourrait également envisager que le réservoir et les moyens d'ouverture se déplacent dans la même direction lors de l'actionnement, le réservoir se déplaçant plus rapidement dans cette direction de sorte qu'il vient en contact avec lesdits moyens d'ouverture pour être ouvert.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation qui comporte avantageusement des moyens 60 déplaçables et/ou déformables sous l'effet de l'inhalation, ces moyens 60 étant adaptés à libérer les moyens de blocage 100. Ces moyens 60 comprennent avantageusement une chambre d'air déformable 61 coopérant avec les moyens de blocage 100 desdits moyens de support mobiles 50. L'inhalation de l'utilisateur provoque la déformation de ladite chambre d'air déformable 61, permettant ainsi de libérer lesdits moyens de blocage 100 et donc de débloquer les moyens de support mobiles 50 pour permettre le déplacement de la roue de guidage 30, et donc du réservoir à vider, vers sa position d'ouverture. Avantageusement, cette chambre d'air 61 peut comprendre une membrane déformable 62, qui peut être reliée d'une part à l'orifice d'inhalation 15 et d'autre part auxdits moyens de blocage 100 de manière directe ou indirecte. Ainsi, lors de l'inhalation, la membrane 62 se déforme et/ou se contracte, provoquant ainsi le déplacement desdits moyens de blocage 100 dans une position de déblocage. Avantageusement, une poche ou diaphragme 62 peut former la chambre d'air 61. Cette poche 62 est reliée à l'orifice d'inhalation 15 via un canal 151 avantageusement disposé autour du canal d'expulsion 152 relié à une chambre de distribution 70. La poche 62 peut être fixée à une tige 101 reliée aux moyens de blocage 100, l'inhalation provoquant la déformation de la poche 62 et donc le pivotement de la tige 101 pour déplacer lesdits moyens de blocage 100. Avantageusement, la poche 62 peut être réalisée en silicone. En variante, la chambre d'air déformable pourrait être réalisée différemment, notamment par une membrane déformable quelconque.

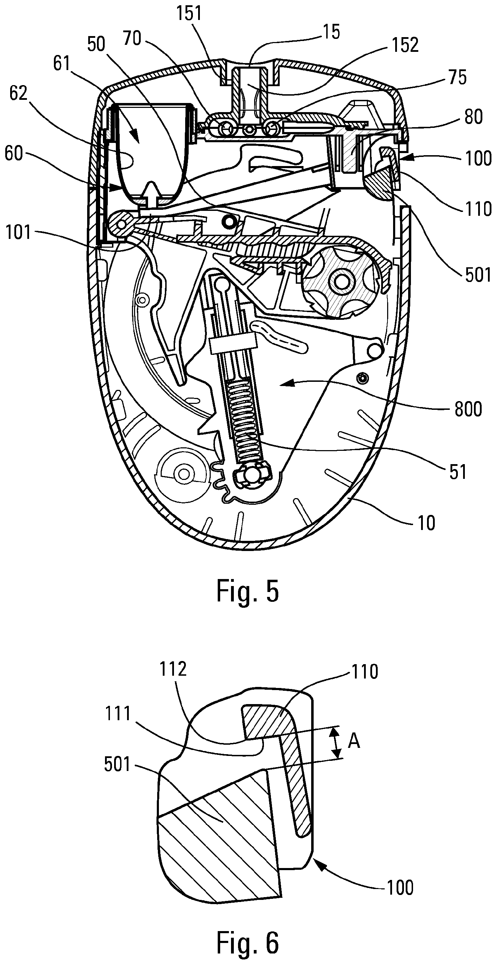

L'inhalateur comporte en outre une chambre de distribution 70 qui est destinée à recevoir la dose de poudre après ouverture d'un réservoir respectif. Avantageusement, cette chambre de distribution 70 est pourvue d'au moins un élément sensiblement sphérique 75, tel qu'une bille, visible sur la figure 5, qui se déplace à l'intérieur de ladite chambre 70 pendant l'inhalation, pour améliorer la distribution du mélange air et poudre après ouverture d'un réservoir, afin d'augmenter l'efficacité du dispositif.

Après l'inhalation, lorsque l'utilisateur referme le dispositif, tous les composants reviennent vers leur position de repos initiale, à savoir que les moyens de support mobiles 50 pivotent par rapport à leur axe de pivotement en retour vers leur position de non distribution en éloignement des moyens d'ouverture de réservoir, et l'élément de chargement est également ramené dans sa position de repos initiale dans laquelle il n'est pas comprimé ou déformé. Le dispositif est alors prêt pour un nouveau cycle d'utilisation.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

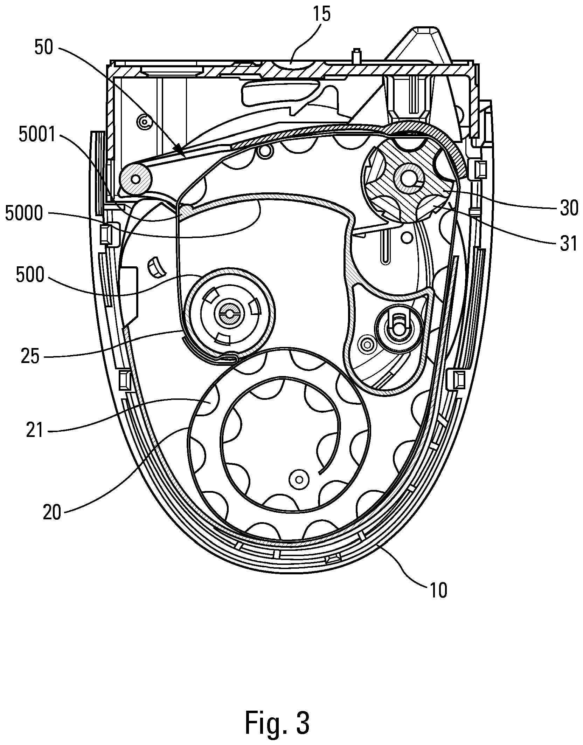

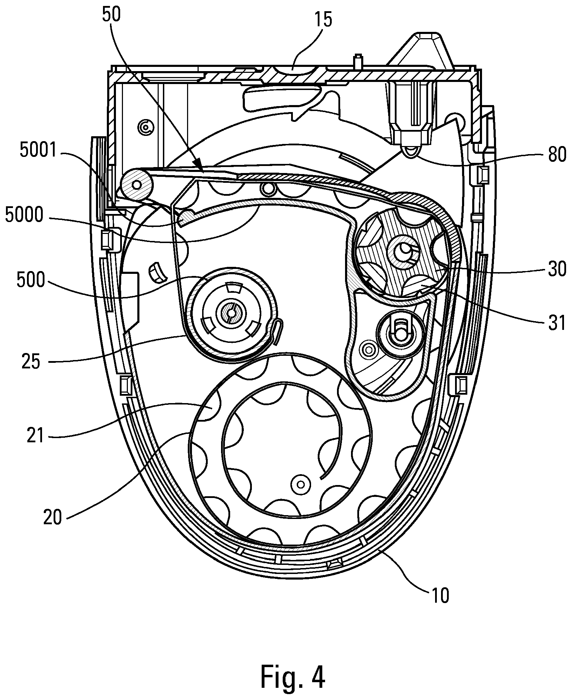

Selon un autre aspect avantageux de l'inhalateur, les réservoirs individuels ou blisters 21 sont formés sur une bande allongée 20, qui est stockée sous forme de bobine à l'intérieur du corps 10 du dispositif. Avantageusement, cette bande de blister enroulée est maintenue par des parois internes dudit corps 10 sans que son extrémité « arrière » (dans le sens de déplacement de la bande de blisters) ne soit fixée par rapport audit corps 10, ce qui permet un assemblage plus aisé de cette bande de blister à l'intérieur du dispositif. La bande de blister est déplacée avantageusement au moyen de la roue de guidage 30 qui présente avantageusement au moins un, de préférence plusieurs évidements 31, visibles sur les figures 3 et 4, dont la forme correspond sensiblement à celle des blisters. Ainsi, lorsque cette roue de guidage 30 tourne, elle entraîne la bande de blister dans la première direction. Bien entendu en variante ou de manière additionnelle, on pourrait utiliser d'autres moyens d'avancée de bande de blisters, par exemple en prévoyant un profil sur les bords longitudinaux latéraux de la bande de blister, ledit profil étant adapté à coopérer avec des moyens d'entraînement appropriés. De même, des trous ménagés le long des bords latéraux de la bande de blister pourraient également être utilisés pour faire avancer la bande de blister au moyen de roues dentées coopérant avec lesdits trous, à la manière d'une pellicule photo.

Avantageusement, l'extrémité avant 25 de la bande de blister 20 est fixée à un élément de réception 500. Pour assurer un bon enroulement de la partie avant de la bande de blisters 20, à savoir celle comportant les blisters vides, cet élément de réception rotatif 500 peut exercer une force de traction sur la bande 20, en particulier sur son extrémité avant 25. Ainsi, on évite tout risque de mauvais enroulement, par exemple de pliage en accordéon ou similaire, de la bande, qui risquerait de bloquer le dispositif. Cette force de traction peut être exercée par un ressort, qui sollicite ledit élément de réception 500 en rotation, et donc tire sur la bande. Ce ressort peut notamment être un ressort à spirale, une ressort à lame ou un ressort hélicoïdal.

Les figures 3 et 4 montrent une variante de réalisation avantageuse, dans laquelle il est prévu un élément de butée 5000 entre la roue d'indexage 30 et l'élément de réception 500. Cet élément de butée a pour but de supprimer, ou tout au moins de diminuer, la force de traction exercée sur la partie de la bande de blisters qui est en contact avec la roue d'indexage 30. En effet, une telle traction, si elle était transmise à ladite roue d'indexage, pourrait solliciter celle-ci en rotation, et donc perturber son bon fonctionnement, par exemple en provoquant un léger décentrage au moment de son actionnement. L'indexation, mais aussi le perçage des blisters, pourraient en être affectés. Avec l'élément de butée 5000, la force de traction sera exercée par l'élément de réception 500 sur l'extrémité avant 25 de la bande, mais ne sera plus appliquée (ou pour le moins à un moindre degré) sur la partie de bande disposée en amont de l'élément de butée 5000 dans le sens d'avancement de la bande 20. Avantageusement, cet élément de butée coopère, de préférence via une extrémité 5001, avec une surface externe de chaque réservoir. Comme visible sur les figures 3 et 4, ces réservoirs ou blisters sont des cavités ayant une surface extérieure arrondie, et l'extrémité 5001 de l'élément de butée 5000 est de préférence aussi arrondie. Lorsque la roue d'indexage 30 ne tourne pas, un réservoir est bloqué par ladite extrémité de l'élément de butée, et la bande ne peut donc pas avancer, ni exercer de traction sur la roue d'indexage 30. Par contre, lorsque la roue d'indexage est tournée, elle exerce une poussée sur la bande, qui combinée avec la traction exercée par l'élément de réception 500, permet de surmonter la butée et de faire passer le réservoir bloqué par-dessus l'élément de butée, de sorte que la bande peut avancer. Avantageusement, la bande de blisters fait un angle au niveau de l'élément de butée, cet angle étant plus grand lorsqu'un réservoir (et donc aussi la roue d'indexage 30) est déplacé contre l'aiguille 80 (figure 3). Comme la roue d'indexage 30 tourne lorsqu'elle est ramenée de la position d'inhalation (figure 3) vers la position de repos (figure 4), cette rotation commence avec l'angle supérieur, ce qui facilite le passage du blister bloqué au-dessus de l'élément de butée 5000. Avantageusement, l'élément de butée 5000 pourrait être déformable ou flexible, pour encore davantage faciliter l'avancée de la bande de blisters lors de l'indexage.

La force de traction exercée par l'élément rotatif 500 sur la bande 20 est totalement indépendante des premiers moyens de déplacement, à savoir la roue d'indexage 30 qui fait avancer la bande lors de chaque actionnement. Ceci permet de garantir que la force de traction ne sera pas dépendante du diamètre d'enroulement de la bande de blisters usée, comme cela serait le cas si la rotation de l'élément rotatif de réception 500 était corrélée à celle de la roue d'indexage 30. Cette force de traction est aussi totalement indépendante des seconds moyens de déplacement formés par les moyens de support mobiles 50, de sorte que l'invention évite de prévoir des moyens d'actionnement relativement complexes pour créer une force de traction sur la bande lors de l'actionnement de l'inhalateur. Ceci simplifie la fabrication et l'assemblage de l'inhalateur.

Selon encore un autre aspect de l'inhalateur, un dispositif de comptage ou d'indication de doses est également prévu. Ce dispositif peut comporter des chiffres ou symboles inscrits directement sur la bande de blister et visibles à travers une fenêtre appropriée 19 dans le corps 10 du dispositif. En variante, on pourrait envisager d'utiliser plusieurs disques rotatifs comportant les chiffres ou symboles, comme cela sera décrit ci-après.

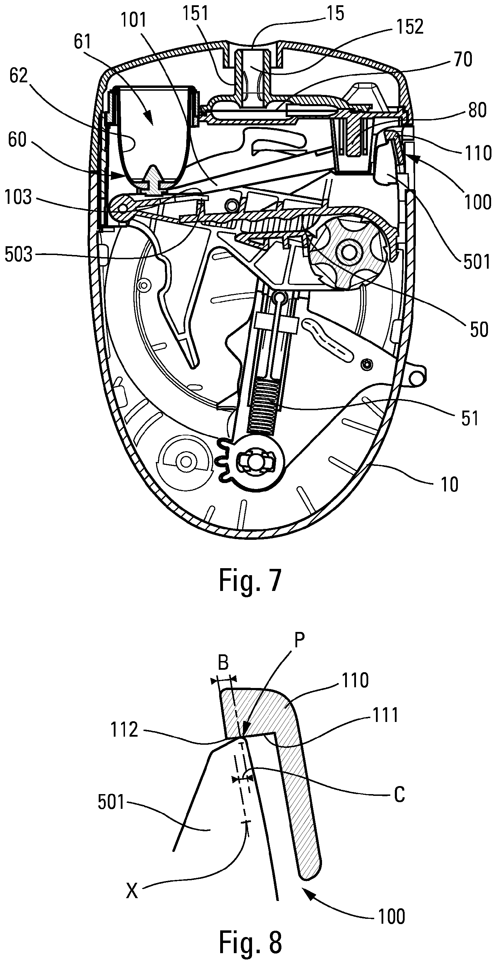

Des moyens de blocage 100 sont prévus pour retenir lesdits moyens de support mobiles 50 dans ladite position de non distribution, correspondant à une position de blocage chargée des moyens de blocage représentée notamment sur la figure 7. Lesdits moyens de blocage 100 comportent avantageusement un élément de blocage 110 adapté à coopérer avec ladite extension ou projection 501 desdits moyens de support mobiles 50. Ledit élément de blocage 110 comporte une surface d'appui 111 sensiblement plane coopérant avec la projection 501, de préférence de forme arrondie, de sorte que ladite projection glisse sur ladite surface d'appui lorsque l'élément de blocage pivote vers sa position de déblocage. Lorsque la projection arrive au niveau d'un bord d'extrémité 112 de la surface d'appui, elle n'est plus retenue, et les moyens de support mobiles 50 peuvent se déplacer vers leur position de distribution sous l'effet de la force exercée sur eux par les moyens de chargement 800. Lesdits moyens de blocage 100 sont avantageusement connectés au diaphragme déformable 62 sensible à l'inhalation de l'utilisateur par l'intermédiaire d'une tige 101, de sorte que lors de l'inhalation de l'utilisateur, ledit diaphragme se déforme, faisant ainsi pivoter la tige 101 et par la même ledit élément de blocage 110, libérant ainsi l'extension 501. Ceci permet le déplacement desdits moyens de support mobiles 50 vers leur position de distribution sous l'effet de la force exercée par le ressort comprimé 51. Ce déplacement des moyens de support mobiles 50 provoque l'ouverture d'un réservoir individuel comme décrit précédemment.

En position de blocage chargée, représentée sur la figure 7, les moyens de support mobiles 50 qui sont sollicités vers la position de distribution par le ressort comprimé 51 exercent donc une force sur les moyens de blocage 100, en particulier sur l'élément de blocage 110 de ces moyens de blocage par l'intermédiaire de l'extension 501. Avantageusement, du côté opposé de la tige 101, à proximité de la connexion de ladite tige 101 au diaphragme 62, il est prévu une zone d'appui 103 adaptée à coopérer avec une zone complémentaire 503 prévue sur les moyens de support mobiles 50. Cette zone d'appui 103 permet de créer une position chargée stable entre lesdits moyens de support mobiles 50 et lesdits moyens de blocage 100. En effet, ces deux moyens sont chacun mobiles et le double contact, avec d'une part une force exercée vers le haut (en se référant à la position représentée sur la figure 7) par l'extension 501 sur la partie d'épaulement 110, et d'autre part une force exercée vers le bas par la zone d'appui 103 sur la zone complémentaire 503, garantit un équilibre de ce blocage qui ne peut être libéré que par l'inhalation de l'utilisateur provoquant la déformation du diaphragme 62 et donc le pivotement de la tige 101 des moyens de blocage 100.

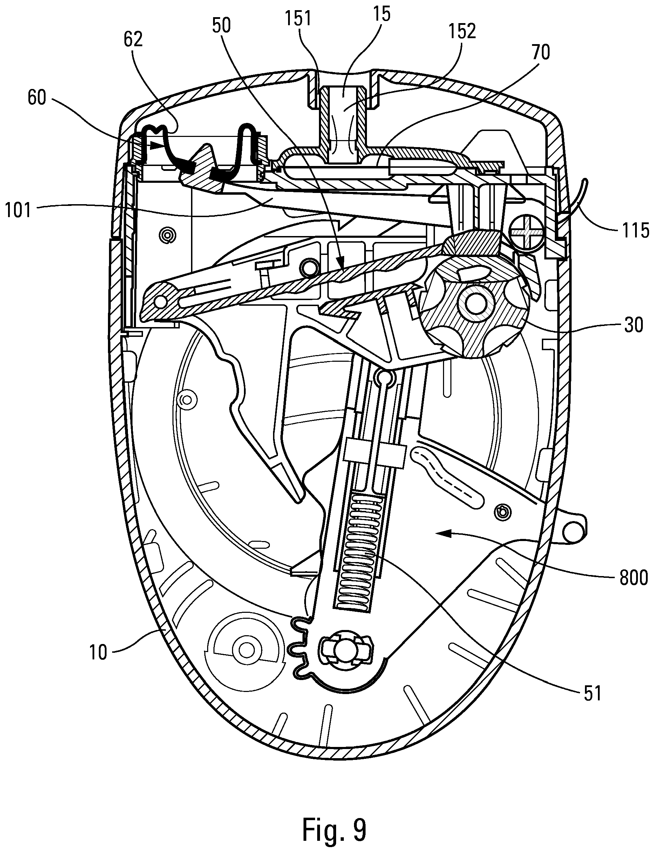

Après l'inhalation, c'est-à-dire en position de distribution représentée sur la figure 9, les moyens de blocage 100 ont pivoté et les moyens de support mobiles 50 ont été déplacés vers le haut par le ressort comprimé 51. Le pivotement des moyens de blocage, en particulier de l'élément de blocage 110, peut provoquer la sortie hors du corps 10 d'une partie d'extrémité 115 de cet élément de blocage 110, comme visible notamment sur la figure 9. Lorsque l'utilisateur referme ensuite les éléments de capot mobile, l'un des éléments de capot vient en fin de fermeture pousser sur ladite partie d'extrémité 115 ce qui ramène les moyens de blocage 100 vers leur position initiale avec le diaphragme 62 qui est également ramené dans sa position initiale, comme visible notamment sur la figure 5.

Ainsi, lorsque l'utilisateur ouvre l'inhalateur il charge le système. S'il n'inhale pas et qu'il referme l'inhalateur, celui-ci reprend simplement sa position de départ sans déplacer les réservoirs 21 ni les moyens de blocage 100. Il n'y a donc aucun risque qu'un réservoir (et donc une dose de produit actif) soit perdu par un actionnement malencontreux ou incomplet dans lequel l'utilisateur n'exercerait pas d'inhalation entre l'ouverture et la fermeture. L'ouverture du réservoir, son vidage, la distribution de la poudre dans les poumons de l'utilisateur ainsi que le déplacement de la bande de blisters pour amener un nouveau réservoir plein en face des moyens d'ouverture n'est donc possible que si l'utilisateur inhale.

Ainsi, les moyens de blocage doivent être mis sous contrainte pour se libérer. Une précharge est donc appliquée sur le mécanisme. L'inhalation du patient provoque la déformation de l'élément déformable 62 (diaphragm) qui entraîne en rotation l'élément de blocage 110 et libère le mécanisme. Un avantage de ces moyens de blocage est de pouvoir fonctionner avec un rapport de 100 entre la précharge appliquée et l'effort nécessaire pour déformer le diaphragme. En effet, 0,05N à 0,2N sont généralement suffisants pour la déformation du diaphragme (effort généré par l'inhalation patient) malgré une précharge de 5 à 10N appliquée sur le mécanisme (cette précharge permet de garantir un effort de perçage suffisant pour permettre à l'aiguille de pénétrer dans le blister 21). Il suffit au patient de générer 4mbar à 15mbar de différentiel de pression (pression correspondant à un débit compris entre 10 litres par minute et 20 litres par minute) pour déclencher le mécanisme: ce débit d'inhalation est un débit jugé confortable pour un patient asthmatique ou atteint d'une maladie chronique de voies pulmonaires. Le débit généré permettra à l'issu du perçage d'entraîner la poudre vers les bronches du patient si le débit minimum d'inhalation n'est pas atteint, aucune dose ne sera libéré: ce dispositif de prévention empêche ainsi le déclenchement intempestif du device.

Un partage judicieux des flux d'air permet de garantir le fonctionnement des moyens de blocage (1/2 à 2/3 du flux global), tout en atteignant les performances de distribution de poudre voulues (1/3 à 1/2 du flux global).

Pour assurer une stabilité des moyens de blocage 100, une valeur d'engagement C (voir figure 8) est prévue pour garantir une tenue suffisante des moyens de blocage préchargée aux vibrations et aux chocs. En effet, un déclenchement intempestif, non voulu et non déclenché par l'inhalation doit être évité. L'engagement C correspond à la distance entre la projection de la force de précharge exercée sur l'élément de blocage 110 au point de contact P entre le projection 501 avec la surface d'appui 111 et l'axe de rotation X de l'élément de blocage 110. Cet engagement C a une valeur non nulle inférieure à 0,5 mm, avantageusement entre 0,2 mm et 0,4 mm. Cet engagement C est couplé avec les butées 103, 503 qui maintiennent le mécanisme préchargé en position d'équilibre stable. L'énergie ainsi stockée sera libérée au moment du perçage après inhalation. Le rôle de ces butées est aussi d'éviter le blocage de la serrure (lors d'un choc ou consécutif à des vibrations): le point de déclenchement reste ainsi identique quelque soit les conditions d'utilisation du device. Une valeur de garde B (voir figure 8) permet d'assurer la synchronisation du déclenchement dans le cycle d'inhalation, pour garantir le bon timing de déclenchement, afin de garder suffisamment de volume d'air pour l'expulsion et la désagglomération de la dose. Elle permet aussi d'ajuster le temps de déclenchement en fonction du profil d'inhalation des patients. La garde B correspond à une distance non nulle (inférieure à 0,5 mm, avantageusement environ 0,2 mm) entre le point de contact P entre la projection 501 avec la surface d'appui 111 et le bord d'extrémité 112 de celle-ci, comme visible sur la figure 8.

Le réarmement des moyens de blocage peut être assuré par un levier 115 (figure 9), ou en variante par un ressort de torsion, ou même par l'élasticité du diaphragme 62. Ce réarmement permet au mécanisme de revenir à son état initial après chaque cycle d'inhalation dès fermeture de l'inhalateur, de manière répétable.

De manière avantageuse, en cas d'ouverture et fermeture du dispositif sans inhalation, le système reste au repos. Il n'y a donc pas de risque de surdosage. De plus, aucune précharge n'est appliquée lorsque l'inhalateur n'est pas ouvert, ce qui favorise la stabilité des composants au cours du temps. Avantageusement, en position fermée de l'inhalateur, la projection 501 est disposée à une distance A non nulle de la surface d'appui 111 pour assurer le réarmement du système.

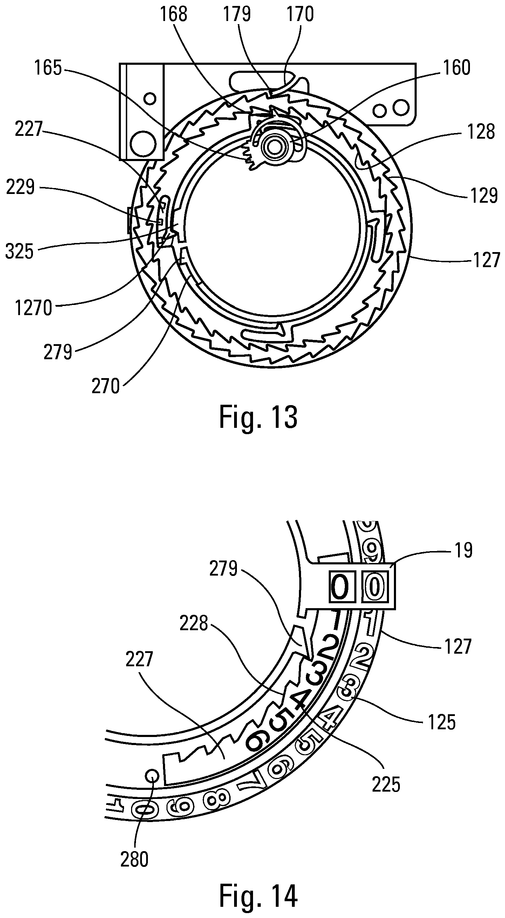

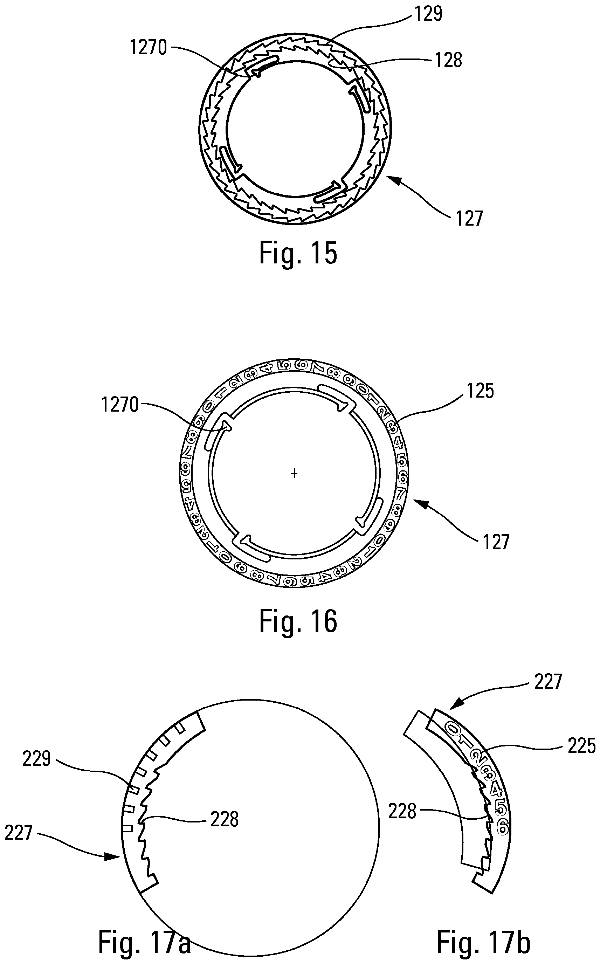

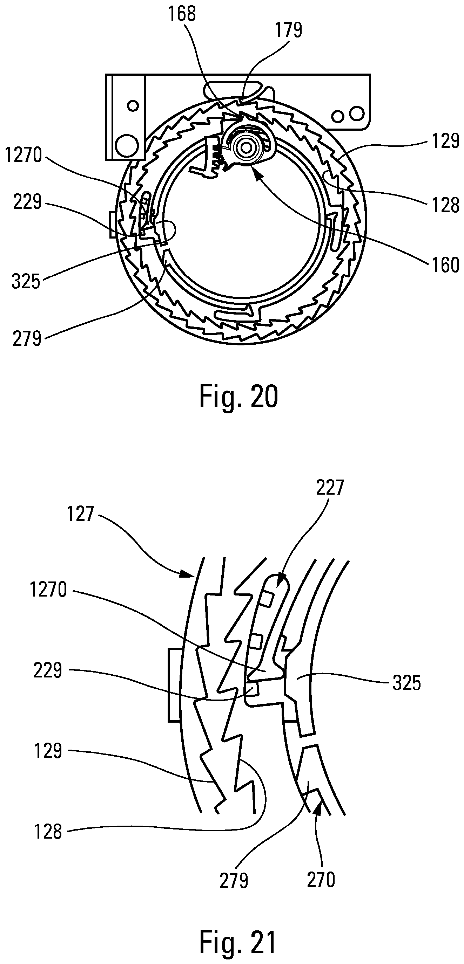

Le dispositif comporte avantageusement un indicateur ou compteur de doses, adapté à compter ou à indiquer à l'utilisateur le nombre de doses distribuées ou restant à distribuer. Dans l'exemple représenté, l'indicateur est adapté à compter 60 doses. Cet indicateur comporte au moins deux éléments de comptage rotatifs, un premier élément de comptage des unités 127 et un second élément de comptage des dizaines 227. Le premier élément de comptage est avantageusement un premier anneau 127 pourvu d'une denture interne (première denture) 128 et d'une denture externe (seconde denture) 129 et comportant des moyens d'indication 125, par exemple des chiffres de 0 à 9, prévus sur une de ses surfaces. De préférence, les moyens d'indication 125 sont disposés sur une surface supérieure, alors que les dentures sont disposées sur une surface inférieure. La denture interne 128 est avantageusement adaptée à coopérer avec un élément d'actionnement ou actionneur 160, alors que la denture externe 129 est avantageusement adaptée à coopérer avec des premiers moyens anti-retour 170, qui sont adaptés à éviter une rotation du premier anneau 127 en sens inverse de celui qui lui est imparti par l'actionneur 160. Le premier anneau 127 comporte au moins un doigt déformable 1270, de préférence quatre doigts répartis à 90° autour de sa périphérie, le ou les doigt(s) déformable(s) étant adapté(s) à coopérer tous les dix actionnements avec une came 325 solidaire du corps. Le second élément de comptage est avantageusement un second anneau, de préférence comme représenté sur les figures 17a et 17b une section angulaire partielle de second anneau 227, notamment une section de 90° ou moins. Cette mise en oeuvre est avantageuse car peu encombrante. Ce second anneau 227 comporte une première denture 228 et une seconde denture 229, ainsi que des seconds moyens d'indication 225 sur une de ses surfaces. Pour un compteur à 60 doses, les seconds moyens d'indication peuvent par exemple comprendre les chiffres 0 à 6. De préférence, le second anneau 227 est concentrique avec le premier anneau 127, les surfaces comportant les moyens d'indication 125, 225 étant coplanaires, le second anneau 227 étant radialement à l'intérieur du premier anneau 127. De cette manière, les premiers et seconds moyens d'indication 125, 225 peuvent s'afficher simultanément dans une fenêtre de visualisation appropriée 19, comme visible sur la figure 14. La denture 229 du second anneau 227 est destinée à coopérer avec le doigt déformable 1270 du premier anneau à chaque fois qu'il est déplacé vers sa position déformée par ladite came 325, comme visible sur les figures 20 et 21. Lorsqu'il n'est pas déformé, ledit doigt 1270 ne coopère pas avec ladite seconde denture 229. Ainsi, dans l'exemple représenté sur les dessins, le premier anneau comporte quatre doigts déformables 1270, ainsi que quatre séries de chiffres 0 à 9 réparties sur la périphérie, les dentures 128, 129 comportant quarante dents. A chaque actionnement, le premier anneau 127 tourne donc de 9°, ce qui correspond à l'avancement d'un chiffre dans la fenêtre 19. Tous les dix actionnements, un doigt déformable 1270 coopère avec ladite came 325, de préférence prévue radialement à l'intérieur dudit doigt, pour être déformé vers l'extérieur et ainsi coopérer avec la seconde denture du seconde anneau. Le second anneau 227 est alors également entraîné en rotation d'un angle de 9°. La première denture 228 du second anneau est destinée à coopérer avec des seconds moyens anti-retour 1270, par exemple une seconde patte flexible 279 qui peut être solidaire du corps. Il est à noter que les premiers et seconds moyens anti-retour 170, 270 pourraient également coopérer avec les mêmes dentures que l'actionneur 160 et le doigt déformable 1270, auquel cas les premier et second anneaux 127, 227 pourraient ne comporter qu'une seule denture chacun. Lorsque le second élément de comptage est une section angulaire d'anneau comme représenté sur les dessins, ses premières et secondes dentures peuvent comporter uniquement le nombre de dents nécessaire, à savoir sept dents pour un compteur de 60 doses (correspondant aux chiffres 0 à 6).

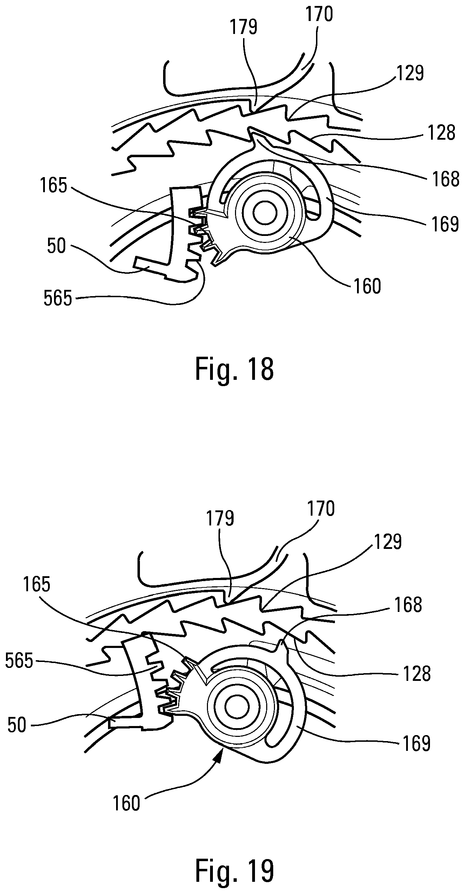

Un but de ce compteur est d'éviter de compter des doses qui ne sont pas distribuées, par exemple en cas d'erreur de manipulation, ou d'une manipulation incomplète du dispositif. Il est donc essentiel que le compteur ou indicateur ne soir actionné qu'une fois que l'utilisateur a inhalé, puisque c'est cette inhalation qui conditionne l'ouverture et la distribution de la dose de chaque blister. Pour ce faire, le dispositif comporte un élément d'actionnement ou actionneur 160 monté pivotant sur le corps 10 entre une première position et une seconde position. Cet actionneur 160 comporte des moyens de connexion 165, notamment un premier engrenage 165 adapté à s'engrener dans un second engrenage 565 (ou des dents complémentaires) prévu sur les moyens de support mobiles 50. Ainsi, lorsque l'utilisateur ouvre le dispositif et qu'il charge les moyens de chargement du dispositif, les moyens de support mobiles 50 ne bougent pas puisqu'ils sont maintenus en position de non-distribution par les moyens de blocage 100. Il ne se passe donc rien au niveau de l'indicateur puisque l'actionneur 160, monté pivotant sur le corps 10 et engrené avec les moyens de support mobiles 50, reste également immobile. Si l'utilisateur referme le dispositif sans inhaler, il ne se passe évidemment rien non plus puisque les moyens de support mobiles 50 restent toujours immobiles. De cette manière, on garantit que l'indicateur ne compte pas de dose s'il n'y a pas d'inhalation. A partir de la position chargée, si l'utilisateur inhale, il provoque le déplacement des moyens de support mobiles 50 dans leur position de distribution en direction des moyens d'ouverture. Ce déplacement provoque donc le pivotement de l'actionneur 160 dans une première direction comme visible sur les figures 18 et 19. L'actionneur 160 comporte un doigt 168 engrené dans la denture interne 128 du premier anneau d'indication 127. Dans cette première direction de déplacement, le doigt 168 de l'actionneur peut glisser sur la pente inclinée de la dent correspondante pour venir se positionner face à la prochaine dent. Parallèlement, les moyens anti-retour 170, en particulier un doigt anti-retour 179, coopèrent avec la denture externe 129 de l'anneau 127 pour empêcher une rotation de celui-ci sous l'effet du frottement, exercé par exemple par le doigt 168 de l'actionneur sur la denture interne 128. Après l'inhalation, lorsque l'utilisateur referme le dispositif, les moyens de support mobiles 50 sont ramenés vers leur position de repos, c'est-à-dire de non-distribution. Ce mouvement provoque donc un pivotement de l'actionneur 160 en sens inverse du précédent, puisque les engrenages respectifs 165, 565 de l'actionneur et des moyens de support mobiles pivotent en sens inverse à celui décrit précédemment. Dans ce déplacement en sens inverse, le doigt 168 de l'actionneur 160 pousse à l'intérieur de la dent dans laquelle il est positionné pour faire tourner le premier anneau 127, comme visible sur la figure 18. Parallèlement, le doigt anti-retour 179 glisse sur la pente inclinée de la dent pour venir se positionner dans la dent suivante de la denture externe 129. En variante, la rotation du premier anneau 127 pourrait avoir lieu lors du déplacement des moyens de support mobiles 50 vers leur position de distribution, par exemple en inversant le sens des dents de la première denture du premier anneau 127.

Un avantage du compteur ci-dessus est qu'il permet l'affichage en grande dimension sans augmenter l'encadrement du compteur et donc de l'inhalateur. En particulier, l'exemple représenté permet pour un compteur de 60 doses, d'afficher des chiffres (unités d'une part, dizaines d'autre part) ayant une hauteur supérieure de 2,5 mm, de préférence environ 2,8 mm, et une largeur supérieure à 1,5 mm, de préférence environ 2,1 mm. Ceci représente une augmentation de la taille des chiffres allant jusqu'à 50% par rapport aux compteurs existants.

Dans l'exemple représenté, l'indicateur est adapté à indiquer le nombre de doses restant à distribuer de sorte que le chiffre affiché diminue à chaque actionnement. L'inverse est bien entendu également possible, à savoir un compteur qui compte le nombre de doses distribuées. Avantageusement, on peut prévoir des moyens de blocage de l'indicateur après distribution de la dernière dose. Ces moyens de blocage peuvent prendre différentes formes, une forme avantageuse étant de prévoir une dent de forme différente sur la denture interne du premier anneau 127 de sorte que l'actionneur ne peut plus venir s'engrener dans la dent suivante pour continuer à faire tourner ledit premier anneau d'indication. D'autres moyens pour bloquer la rotation du premier anneau après distribution de la dernière dose sont également envisageables. Par exemple, une butée 280 pour le second anneau des dizaines 227, comme représenté schématiquement sur la figure 14.

Dans tous les modes de réalisation décrits ci-dessus, la bande de blister est formée par une bande présentant deux extrémités. En variante, on pourrait utiliser une bande continue. D'autres modifications sont également possibles sans sortir du cadre de la présente invention.

La présente invention permet donc de fournir un inhalateur de poudre sèche qui procure notamment les fonctions suivantes :
▪ une pluralité de doses individuelles de poudre stockées dans des réservoirs individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération préchargé ;
▪ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
▪ des moyens pour éviter une perte de dose en cas d'ouverture de l'inhalateur mais en l'absence d'inhalation. Dans ce cas, lors de la fermeture de l'inhalateur, le dispositif revient exactement à sa position de départ ;
▪ un indicateur de doses adapté à compter les doses seulement en cas d'inhalation.

D'autres fonctions sont également fournies par le dispositif de l'invention tel que cela a été décrit précédemment. Il est à noter que les différentes fonctions, même si elles ont été représentées comme prévues simultanément sur les différents modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les réservoirs individuels sont arrangés les uns par rapport aux autres, etc. Les moyens d'armement et le système de déclenchement par l'inhalation pourraient être réalisés différemment. Il en est de même des autres parties constitutives du dispositif.

L'inhalateur de l'invention, incorporant toutes ou parties des fonctions décrites, s'avère fournir des performances supérieures à celles des dispositifs existants. En particulier, l'inhalateur de l'invention fournit de préférence un taux de vidage des réservoirs d'au moins 90% à chaque actionnement. Avantageusement, ce taux de vidage, correspondant au pourcentage du produit fluide expulsé hors d'un réservoir ouvert lors d'un actionnement du dispositif, est supérieur à 95%, de préférence même supérieur à 97%. Ce taux de vidage élevé est notamment même supérieur aux performances obtenues avec des inhalateurs actifs qui sont généralement plus efficaces que les inhalateurs passifs, et dans lesquels ce n'est pas l'écoulement d'inhalation qui vide le blister et expulse la dose mais un écoulement d'air comprimé libéré au moment de l'inhalation. Il garantit une efficacité maximale du dispositif de l'invention. Couplé à l'ouverture déclenchée par l'inhalation, ce taux de vidage élevé garantit une distribution optimale du produit fluide, en l'occurrence la poudre, dans les poumons de l'utilisateur. Par ailleurs, l'invention fournit également une meilleure régularité de vidage des réservoirs lors d'actionnements successifs. Ainsi, en se référant par exemple à 10 réservoirs d'une bande de blisters, il s'avère que le taux de vidage varie de moins de 15%, avantageusement de moins de 10%, de préférence de moins de 5% d'un réservoir à l'autre. Cette meilleure régularité garantit une meilleure reproductibilité de la dose et donc aussi une meilleure efficacité du dispositif de l'invention.

Diverses modifications sont également possibles pour un homme du métier sans sortir du cadre de la présente invention tel que défini par les revendications annexées. En particulier, les diverses caractéristiques et fonctionnalités du dispositif décrites en référence aux différents modes de réalisation et variantes peuvent être adaptées à tous ces modes de réalisation et variantes, et peuvent être combinées entre elles d'une quelconque façon appropriée.

## Revendications

1. Dispositif de distribution de produits fluides, comportant un corps (10) pourvu d'un orifice de distribution (15), ledit dispositif comportant en outre une bande souple allongée (20) supportant une pluralité de réservoirs (21) contenant chacun une dose de produit fluide, des moyens d'ouverture de réservoir (80) pour ouvrir un réservoir respectif à chaque actionnement, des premiers moyens de déplacement (30) pour faire avancer ladite bande souple (20) avant et/ou pendant et/ou après chaque actionnement, pour amener un réservoir plein face auxdits moyens d'ouverture de réservoir, et des seconds moyens de déplacement (50) pour déplacer un réservoir plein (21) contre lesdits moyens d'ouverture (80) à chaque actionnement du dispositif, ledit dispositif comportant des moyens de positionnement (300) adaptés à précisément positionner le réservoir plein par rapport aux moyens d'ouverture de réservoir (80) lorsqu'il est déplacé contre eux, afin de garantir une ouverture prédéterminée et reproductible à chaque actionnement, lesdits moyens de positionnement (300) comportant une projection (301) solidaire des moyens d'ouverture (80), **caractérisé en ce que** ladite projection (301) est tronconique et est adaptée à coopérer avec des encoches complémentaires (38) formées sur lesdits premiers moyens de déplacement (30).

2. Dispositif selon la revendication 1, dans lequel les moyens de positionnement (300) sont adaptés à prédéterminer la position angulaire desdits premiers moyens de déplacement (300).

3. Dispositif selon l'une quelconque des revendications précédentes, dans lequel les moyens de positionnement (300) sont adaptés à former une butée pour les seconds moyens de déplacement (50).

4. Dispositif selon la revendication 3, dans lequel lesdits moyens d'ouverture (80) comportent une aiguille adaptée à pénétrer dans le réservoir plein (21), lesdits moyens de positionnement définissant la profondeur de pénétration de l'aiguille dans le réservoir.

5. Dispositif selon l'une quelconque des revendications précédentes, dans lequel lesdits seconds moyens de déplacement (50) sont déclenchés par l'inhalation de l'utilisateur.

## Patentansprüche

1. Ausgabevorrichtung für fluide Produkte, aufweisend einen mit einer Ausgabeöffnung (15) versehenen Körper (10), wobei die Vorrichtung des Weiteren einen flexiblen, langgestreckten Streifen (20), der mehrere Behälter (21) trägt, die jeweils eine Dosis fluides Produkt enthalten, Behälteröffnungsmittel (80) zum Öffnen eines jeweiligen Behälters bei jeder Betätigung, erste Verschiebemittel (30) zum Vorschieben des flexiblen Streifens (20) vor und/oder während und/oder nach jeder Betätigung, um einen vollen Behälter gegenüber den Behälteröffnungsmitteln anzuordnen, und zweite Verschiebemittel (50) zum Verschieben eines vollen Behälters (21) gegen die Öffnungsmittel (80) bei jeder Betätigung der Vorrichtung aufweist, wobei die Vorrichtung Positionierungsmittel (300) aufweist, die dazu geeignet sind, den vollen Behälter in Bezug auf die Behälteröffnungsmittel (80) genau zu positionieren, wenn er gegen diese verschoben wird, um eine vorbestimmte und reproduzierbare Öffnung bei jeder Betätigung zu gewährleisten, wobei die Positionierungsmittel (300) einen mit den Öffnungsmitteln (80) einstückigen Vorsprung (301) aufweisen, **dadurch gekennzeichnet, dass** der Vorsprung (301) kegelstumpfartig und dafür geeignet ist, mit komplementären Kerben (38) zusammenzuwirken, die auf den ersten Verschiebemitteln (30) ausgebildet sind.

2. Vorrichtung nach Anspruch 1, wobei die Positionierungsmittel (300) dazu geeignet sind, die Winkelposition der ersten Verschiebemittel (300) vorzubestimmen.

3. Vorrichtung nach einem der vorhergehenden Ansprüche, wobei die Positionierungsmittel (300) dazu geeignet sind, einen Anschlag für die zweiten Verschiebemittel (50) zu bilden.

4. Vorrichtung nach Anspruch 3, wobei die Öffnungsmittel (80) eine Nadel aufweisen, die dazu geeignet ist, in den vollen Behälter (21) einzudringen, wobei die Positionierungsmittel die Eindringtiefe der Nadel in den Behälter definieren.

5. Vorrichtung nach einem der vorhergehenden Ansprüche, wobei die zweiten Verschiebemittel (50) durch Inhalation durch den Benutzer ausgelöst werden.

## Claims

1. A fluid dispenser device comprising a body (10) provided with a dispenser orifice (15), said device further comprising: an elongate flexible strip (20) supporting a plurality of reservoirs (21) each containing a dose of fluid; reservoir-opening means (80) for opening a respective reservoir on each actuation; first displacement means (30) for causing said flexible strip (20) to advance before and/or during and/or after each actuation, so as to bring a full reservoir into register with said reservoir-opening means; and second displacement means (50) for displacing a full reservoir (21) against said opening means (80) each time the device is actuated, said device including positioning means (300) that are adapted to position the full reservoir accurately relative to the reservoir-opening means (80) when it is displaced thereagainst, so as to guarantee that opening is predetermined and reproducible on each actuation, said positioning means (300) comprising a projection (301) that is secured to the opening means (80), **characterized in that** said projection (301) is frustoconical and is adapted to co-operate with complementary notches (38), which are formed on said first displacement means (30).

2. A device according to claim 1, wherein the positioning means (300) are adapted to predetermine the angular position of said first displacement means (300).

3. A device according to any preceding claim, wherein the positioning means (300) are adapted to form an abutment for the second displacement means (50).

4. A device according to claim 3, wherein said opening means (80) comprise a needle that is adapted to penetrate into the full reservoir (21), said positioning means defining the depth to which the needle penetrates into the reservoir.

5. A device according to any preceding claim, wherein said second displacement means (50) are triggered by the user inhaling.
